(19) Europäisches Patentamt
European Patent Office
Office européen des brevets

(11) Veröffentlichungsnummer: 0 100 425
A1

(12) # EUROPÄISCHE PATENTANMELDUNG

(21) Anmeldenummer: 83106072.8

(22) Anmeldetag: 22.06.83

(51) Int. Cl.³: C 07 C 125/08
A 01 N 47/40

(30) Priorität: 03.07.82 DE 3224984

(43) Veröffentlichungstag der Anmeldung:
15.02.84 Patentblatt 84/7

(84) Benannte Vertragsstaaten:
AT BE CH DE FR GB IT LI NL

(71) Anmelder: BAYER AG
Zentralbereich Patente, Marken und Lizenzen
D-5090 Leverkusen 1, Bayerwerk(DE)

(72) Erfinder: Führer, Wolfgang, Dr.
Wehrstrasse 28
D-5202 Hennef 1(DE)

(72) Erfinder: Stetter, Jörg, Dr.
Gellertweg 4
D-5600 Wuppertal 1(DE)

(72) Erfinder: Eue, Ludwig, Dr.
Paul-Klee-Strasse 36
D-5090 Leverkusen(DE)

(72) Erfinder: Schmidt, Robert R.,Dr.
Im Waldwinkel 110
D-5060 Bergisch-Gladbach 2(DE)

(54) **Substituierte Diphenylether, Verfahren zu ihren Herstellung und ihre Verwendung als Herbizide.**

(57) Neue substituierte Diphenylether der Formel

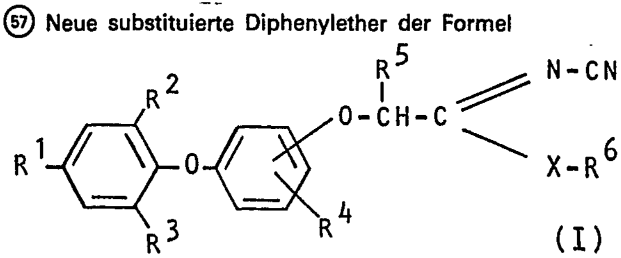

(I)

in welcher

$R^1$ für Halogen oder Trifluormethyl steht,

$R^2$ für Wasserstoff, Halogen, Trifluormethyl oder Nitro steht,

$R^3$ für Wasserstoff oder Halogen steht,

$R^4$ für Wasserstoff, Halogen oder Nitro steht,

$R^5$ für Wasserstoff oder Alkyl mit 1 bis 5 Kohlenstoffatomen steht,

$R^6$ für Alkyl, Alkenyl, Alkinyl oder Alkoxyalkyl steht und

X für Sauerstoff oder Schwefel steht,

mehrere Verfahren zur Herstellung der erfindungsgemäßen Stoffe und deren Verwendung als Herbizide.

Neue Ausgangsstoffe zut Herstellung der erfindungsgemäßen Verbindungen und die Synthese dieser neuen Ausgangsstoffe.

EP 0 100 425 A1

Croydon Printing Company Ltd.

BAYER AKTIENGESELLSCHAFT 5090 Leverkusen, Bayerwerk
Zentralbereich
Patente, Marken und Lizenzen Dü/bo/c
Ib

# Substituierte Diphenylether, Verfahren zu ihrer Herstellung und ihre Verwendung als Herbizide

Die Erfindung betrifft neue substituierte Diphenylether, mehrere Verfahren zu ihrer Herstellung sowie ihre Verwendung als Herbizide.

Es ist bereits bekannt, daß bestimmte substituierte Diphenylether herbzide Eigenschaften besitzen (vgl. DE-OS 2 613 697). Die Wirkung dieser Stoffe ist jedoch nicht immer ausreichend.

Es wurden nun neue substituierte Diphenylether der Formel

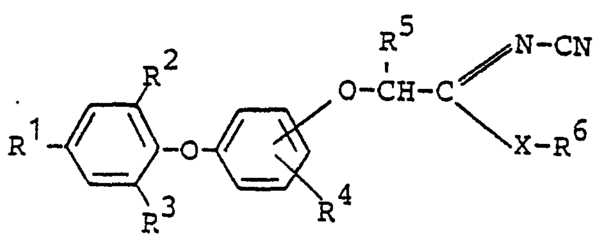

(I)

in welcher

$R^1$ für Halogen oder Trifluormethyl steht,

$R^2$ für Wasserstoff, Halogen, Trifluormethyl oder Nitro steht,

$R^3$ für Wasserstoff oder Halogen steht,

$R^4$ für Wasserstoff, Halogen oder Nitro steht,

$R^5$ für Wasserstoff oder Alkyl mit 1 bis 5 Kohlenstoffatomen steht,

$R^6$ für Alkyl, Alkenyl, Alkinyl oder Alkoxyalkyl steht und

X für Sauerstoff oder Schwefel steht,

gefunden.

Die Verbindungen der Formel (I) können in der syn- oder anti-Form oder auch in Form von Gemischen aus syn- und anti-Form vorliegen. Außerdem können diejenigen Verbindungen der Formel (I), die eines oder mehrere asymmetrische Kohlenstoffatome enthalten, in Form von Racematen oder verschiedenen optischen Isomeren auftreten. Die Erfindung betrifft sowohl die geometrischen als auch die optischen Isomeren und deren Gemische.

Weiterhin wurde gefunden, daß man neue substituierte Diphenylether der Formel (I) erhält, wenn man

a) Iminoester der Formel

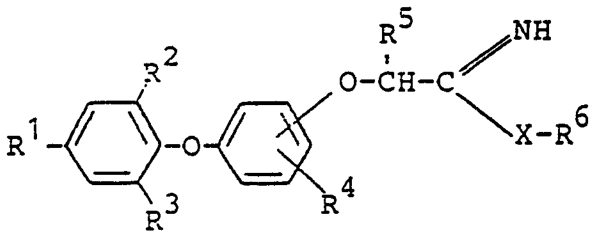

(II)

in welcher

$R^1, R^2, R^3, R^4, R^5, R^6$ und X die oben angegebene Bedeutung haben,

oder Hydrohalogenide der Iminoester der Formel (II) mit Cyanamid der Formel

$H_2N-CN$ (III)

gegebenenfalls in Gegenwart eines Säureakzeptors und gegebenenfalls in Gegenwart eines Verdünnungsmittels umsetzt,

oder

b) Phenoxy-phenol-Derivate der Formel

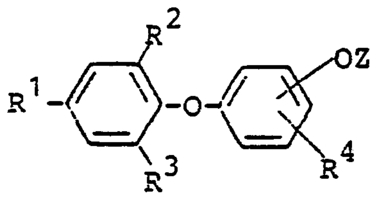

(IV)

in welcher

$R^1$, $R^2$, $R^3$ und $R^4$ die oben angegebene Bedeutung haben und

Z für Wasserstoff oder ein Äquivalent eines Alkalimetalles oder Erdalkalimetalles steht,

mit Verbindungen der Formel

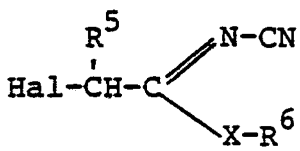

(V)

in welcher

$R^5$, $R^6$ und X die oben angegebene Bedeutung haben und

Hal für Chlor oder Brom steht,

gegebenenfalls in Gegenwart eines Säureakzeptors und gegebenenfalls in Gegenwart eines Verdünnungsmittels umsetzt,

oder

c) Halogenbenzole der Formel

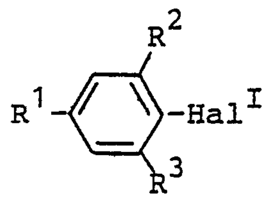

(VI)

in welcher

$R^1$, $R^2$ und $R^3$ die oben angegebene Bedeutung haben und

$Hal^I$ für Chlor oder Brom steht,

mit Phenol-Derivaten der Formel

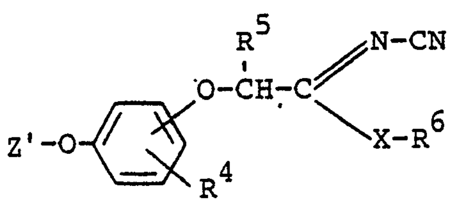

(VII)

in welcher

$R^4$, $R^5$, $R^6$ und X die oben angegebene Bedeutung haben und

Z' für Wasserstoff oder ein Äquivalent eines Alkalimetalles oder eines Erdalkalimetalles steht,

gegebenenfalls in Gegenwart eines Säureakzeptors und gegebenenfalls in Gegenwart eines Verdünnungsmittels umsetzt,

oder

d) Phenol-Derivate der Formel

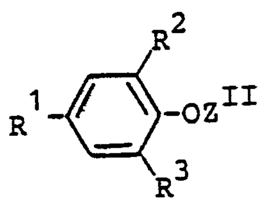

(VIII)

in welcher

$R^1$, $R^2$ und $R^3$ die oben angegebene Bedeutung haben und

$Z^{II}$ für Wasserstoff oder ein Äquivalent eines Alkalimetalles oder eines Erdalkalimetalles steht,

mit Verbindungen der Formel

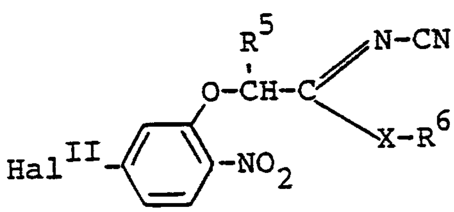

(IX)

in welcher

$R^5$, $R^6$ und X die oben angegebene Bedeutung haben und

$Hal^{II}$ für Chlor oder Brom steht,

gegebenenfalls in Gegenwart eines Säureakzeptors und gegebenenfalls in Gegenwart eines Verdünnungsmittels umsetzt,

oder

e) Verbindungen der Formel

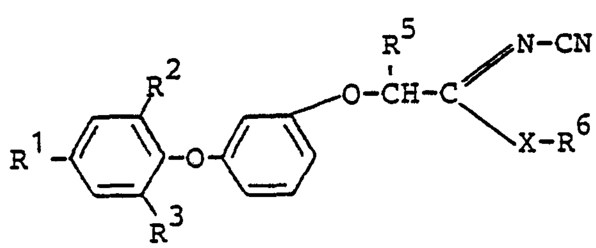

(Ia)

in welcher

$R^1$, $R^2$, $R^3$, $R^5$, $R^6$ und X die oben angegebene Bedeutung haben,

mit einem Nitrierungsmittel, gegebenenfalls in Gegenwart eines Katalysators sowie gegebenenfalls in Gegenwart eines Verdünnungsmittels umsetzt.

Schließlich wurde gefunden, daß sich die neuen substituierten Diphenylether der Formel (I) durch hervorragende herbizide Eigenschaften auszeichnen.

Überraschenderweise besitzen die erfindungsgemäßen substituierten Diphenylether der Formel (I) eine bessere herbizide Wirksamkeit als konstitutionell ähnliche vorbekannte Wirkstoffe gleicher Wirkungsrichtung.

Die erfindungsgemäßen substituierten Diphenylether sind durch die Formel (I) allgemein definiert. In dieser Formel stehen vorzugsweise

$R^1$ für Fluor, Chlor, Brom oder Trifluormethyl,

$R^2$ für Wasserstoff, Fluor, Chlor, Brom, Trifluormethyl oder Nitro,

$R^3$ für Wasserstoff, Fluor, Chlor oder Brom,

$R^4$ für Wasserstoff, Fluor, Chlor, Brom oder Nitro,

$R^5$ für Wasserstoff oder Alkyl mit 1 bis 4 Kohlenstoffatomen,

$R^6$ für Alkyl mit 1 bis 6 Kohlenstoffatomen, Alkenyl mit 2 bis 6 Kohlenstoffatomen, Alkinyl mit 2 bis 6 Kohlenstoffatomen, Alkoxyalkyl mit 1 bis 4 Kohlenstoffatomen im Alkoxyteil und 1 bis 4 Kohlenstoffatomen im Alkylteil und

X für Sauerstoff oder Schwefel.

Bevorzugt sind außerdem die entsprechenden geometrischen und optischen Isomeren.

Als Beispiele für die erfindungsgemäßen Stoffe der Formel (I) seien im einzelnen die in den nachfolgenden Tabellen 1 und 2 formelmäßig aufgeführten Stoffe genannt.

Tabelle 1

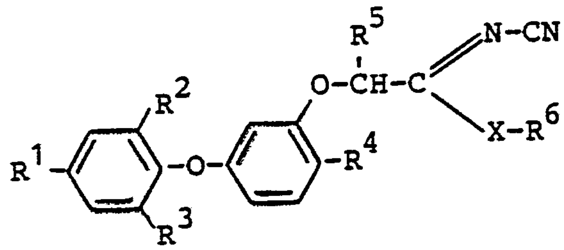

(Ib)

| $R^1$ | $R^2$ | $R^3$ | $R^4$ | $R^5$ | $R^6$ | X |
|---|---|---|---|---|---|---|
| $CF_3$ | H | H | H | H | $CH_3$ | O |
| $CF_3$ | H | H | H | H | $CH_3$ | S |
| $CF_3$ | H | H | $NO_2$ | H | $CH_3$ | O |
| $CF_3$ | H | H | $NO_2$ | H | $CH_3$ | S |
| Cl | $CF_3$ | H | $NO_2$ | H | $CH_3$ | O |
| Cl | $CF_3$ | H | $NO_2$ | H | $CH_3$ | S |
| $CF_3$ | H | H | H | H | $C_2H_5$ | O |
| $CF_3$ | H | H | H | H | $C_2H_5$ | S |
| $CF_3$ | H | H | $NO_2$ | H | $C_2H_5$ | O |
| $CF_3$ | H | H | $NO_2$ | H | $C_2H_5$ | S |
| $CF_3$ | H | H | $NO_2$ | H | $C_3H_7$-n | O |
| $CF_3$ | H | H | $NO_2$ | H | $C_3H_7$-n | S |
| Cl | $CF_3$ | H | $NO_2$ | H | $C_2H_5$ | O |
| Cl | $CF_3$ | H | $NO_2$ | H | $C_2H_5$ | S |
| $CF_3$ | Cl | H | H | H | $CH_3$ | O |
| $CF_3$ | Cl | H | H | H | $CH_3$ | S |
| $CF_3$ | Cl | H | $NO_2$ | H | $CH_3$ | O |
| $CF_3$ | Cl | H | $NO_2$ | H | $CH_3$ | S |
| $CF_3$ | Cl | Cl | H | H | $CH_3$ | O |
| $CF_3$ | Cl | Cl | H | H | $CH_3$ | S |

Tabelle 1 (Fortsetzung)

| $R^1$ | $R^2$ | $R^3$ | $R^4$ | $R^5$ | $R^6$ | X |
|---|---|---|---|---|---|---|
| $CF_3$ | Cl | Cl | $NO_2$ | H | $CH_3$ | O |
| $CF_3$ | Cl | Cl | $NO_2$ | H | $CH_3$ | S |
| $CF_3$ | H | H | H | $CH_3$ | $CH_3$ | O |
| $CF_3$ | H | H | H | $CH_3$ | $CH_3$ | S |
| $CF_3$ | H | H | $NO_2$ | $CH_3$ | $CH_3$ | O |
| $CF_3$ | H | H | $NO_2$ | $CH_3$ | $CH_3$ | S |
| Cl | $CF_3$ | H | $NO_2$ | $CH_3$ | $CH_3$ | O |
| Cl | $CF_3$ | H | $NO_2$ | $CH_3$ | $CH_3$ | S |
| $CF_3$ | H | H | H | $CH_3$ | $C_2H_5$ | O |
| $CF_3$ | H | H | H | $CH_3$ | $C_2H_5$ | S |
| $CF_3$ | H | H | $NO_2$ | $CH_3$ | $C_2H_5$ | O |
| $CF_3$ | H | H | $NO_2$ | $CH_3$ | $C_2H_5$ | S |
| Cl | $CF_3$ | H | $NO_2$ | $CH_3$ | $C_2H_5$ | O |
| Cl | $CF_3$ | H | $NO_2$ | $CH_3$ | $C_2H_5$ | S |
| $CF_3$ | Cl | H | H | $CH_3$ | $CH_3$ | O |
| $CF_3$ | Cl | H | H | $CH_3$ | $CH_3$ | S |
| $CF_3$ | Cl | H | $NO_2$ | $CH_3$ | $CH_3$ | O |
| $CF_3$ | Cl | H | $NO_2$ | $CH_3$ | $CH_3$ | S |
| $CF_3$ | Cl | Cl | H | $CH_3$ | $CH_3$ | O |
| $CF_3$ | Cl | Cl | H | $CH_3$ | $CH_3$ | S |
| $CF_3$ | Cl | Cl | $NO_2$ | $CH_3$ | $CH_3$ | O |
| $CF_3$ | Cl | Cl | $NO_2$ | $CH_3$ | $CH_3$ | S |

Tabelle 2

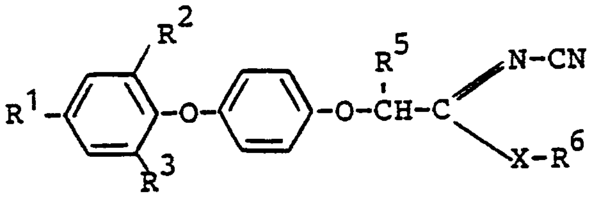

(Ic)

| $R^1$ | $R^2$ | $R^3$ | $R^5$ | $R^6$ | X |
|---|---|---|---|---|---|
| $CF_3$ | H | H | H | $CH_3$ | O |
| $CF_3$ | H | H | H | $CH_3$ | S |
| Cl | $CF_3$ | H | H | $CH_3$ | O |
| Cl | $CF_3$ | H | H | $CH_3$ | S |
| $CF_3$ | H | H | H | $C_2H_5$ | O |
| $CF_3$ | H | H | H | $C_2H_5$ | S |
| Cl | $CF_3$ | H | H | $C_2H_5$ | O |
| Cl | $CF_3$ | H | H | $C_2H_5$ | S |
| $CF_3$ | Cl | H | H | $CH_3$ | O |
| $CF_3$ | Cl | H | H | $CH_3$ | S |
| $CF_3$ | Cl | Cl | H | $CH_3$ | O |
| $CF_3$ | Cl | Cl | H | $CH_3$ | S |
| $CF_3$ | H | H | $CH_3$ | $CH_3$ | O |
| $CF_3$ | H | H | $CH_3$ | $CH_3$ | S |
| Cl | $CF_3$ | H | $CH_3$ | $CH_3$ | O |
| Cl | $CF_3$ | H | $CH_3$ | $CH_3$ | S |
| $CF_3$ | H | H | $CH_3$ | $C_2H_5$ | O |
| $CF_3$ | H | H | $CH_3$ | $C_2H_5$ | S |
| $CF_3$ | Cl | H | $CH_3$ | $C_2H_5$ | O |
| $CF_3$ | Cl | H | $CH_3$ | $C_2H_5$ | S |
| $CF_3$ | Cl | Cl | $CH_3$ | $C_2H_5$ | O |
| $CF_3$ | Cl | Cl | $CH_3$ | $C_2H_5$ | S |

Verwendet man 2-/4-(2,6-Dichlor-4-trifluormethyl-phenoxy)-phenoxy7-imino-propionsäure-methylester und Cyanamid als Ausgangsstoffe, so kann der Verlauf des erfindungsgemäßen Verfahrens (a) durch das folgende Formelschema wiedergegeben werden:

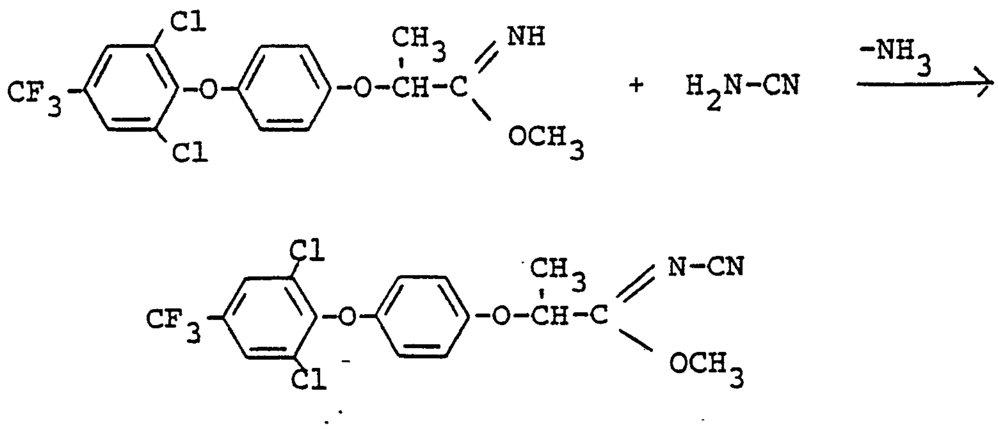


Verwendet man 4-(4-Trifluormethyl-phenoxy)-phenol und 2-Brom-1-cyanimino-propionsäure-ethylester als Ausgangsstoffe, so kann der Verlauf des erfindungsgemäßen Verfahrens (b) durch das folgende Formelschema wiedergegeben werden:

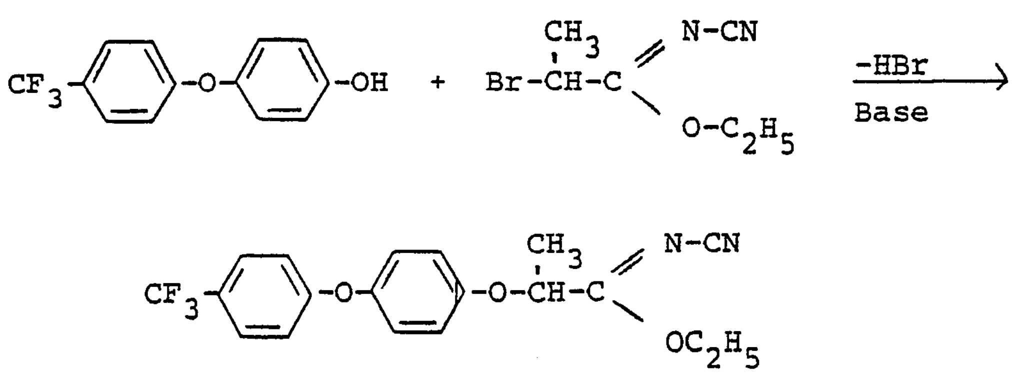

Verwendet man 2-Brom-5-trifluormethyl-chlorbenzol und 2-(3-Hydroxy-phenoxy)-1-cyanimino-propionsäure-ethylester als Ausgangsstoffe, so kann der Verlauf des erfindungsgemäßen Verfahrens (c) durch das folgende Formelschema wiedergegeben werden:

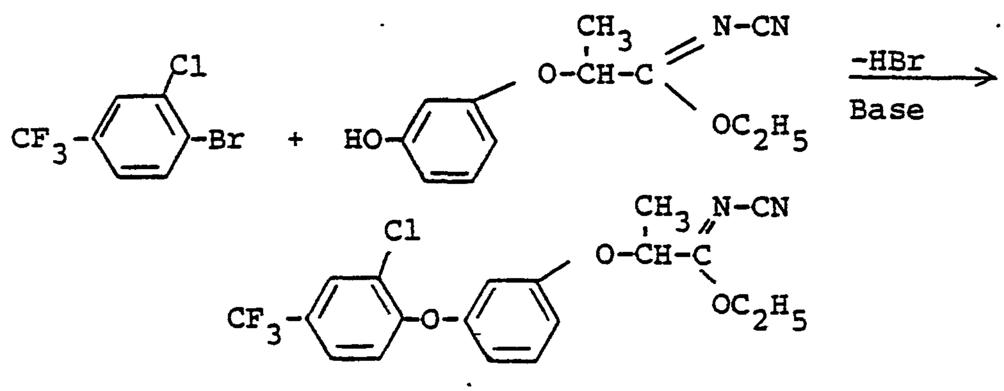


Verwendet man 2-Chlor-4-trifluormethyl-natrium-phenolat und 2-(5-Brom-2-nitrophenoxy)-1-cyanimino-propionsäure-methylester als Ausgangsstoffe, so kann der Verlauf des erfindungsgemäßen Verfahrens (d) durch das folgende Formelschema wiedergegeben werden:

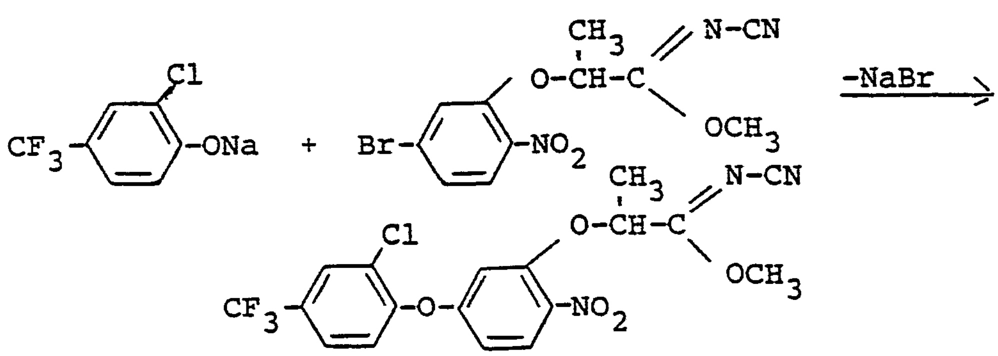

Verwendet man 2-/3-(2,6-Dichlor-4-trifluormethyl-phenoxy)-phenoxy/-1-cyanimino-propionsäure-ethylester als Ausgangsstoff und konzentrierte Salpetersäure als Nitrierungsmittel, so kann der Verlauf des erfindungsgemäßen Verfahrens (e) durch das folgende Formelschema wiedergegeben werden:

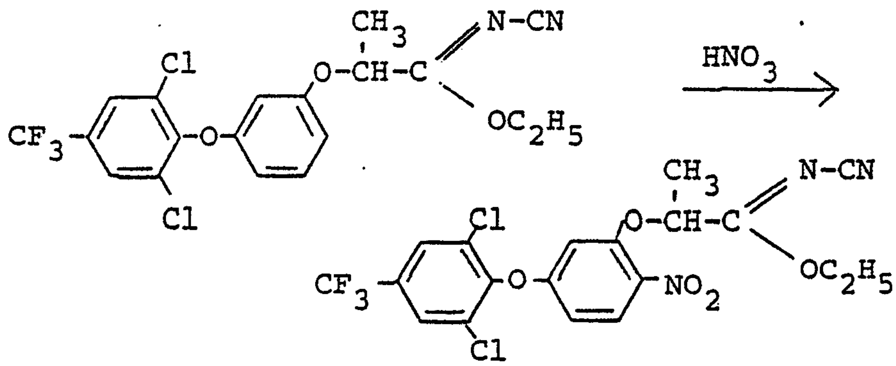


Die bei dem erfindungsgemäßen Verfahren (a) als Ausgangsstoffe benötigten Iminoester sind durch die Formel (II) definiert. In dieser Formel haben $R^1$, $R^2$, $R^3$, $R^4$, $R^5$, $R^6$ und X vorzugsweise diejenigen Bedeutungen, die bereits im Zusammenhang mit der Beschreibung der erfindungsgemäßen Stoffe der Formel (I) vorzugsweise für diese Reste genannt wurden.

Die Verbindungen der Formel (II) sind bisher noch nicht bekannt. Sie lassen sich jedoch nach im Prinzip bekannten Verfahren in einfacher Weise herstellen. So erhält man die Iminoester der Formel (II), indem man Nitrile der Formel

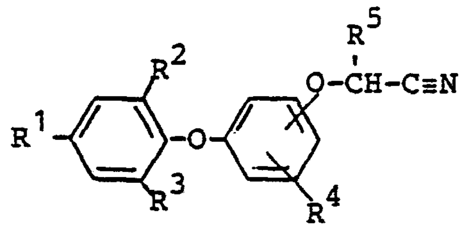

(X)

in welcher

$R^1$, $R^2$, $R^3$, $R^4$ und $R^5$ die oben angegebene Bedeutung haben,

mit Verbindungen der Formel

$$H\text{-}X\text{-}R^6 \qquad (XI)$$

in welcher

$R^6$ und X die oben angegebene Bedeutung haben,

in Gegenwart von Chlorwasserstoff und in Gegenwart eines Verdünnungsmittels, wie beispielsweise Toluol, bei Temperaturen zwischen -10°C und +30°C umsetzt (vgl. DE-OS 2 746 057 und Ber. Dtsch. Chem. Ges. 16, 356 (1883)).

Die Verbindungen der Formel (X) sind teilweise bekannt (vgl. DE-OS 2 613 697). Sie können erhalten werden, indem man Verbindungen der Formel (IV) mit entsprechenden Halogen-Nitrilen in Gegenwart eines inerten organischen Lösungsmittels, wie z.B. Acetonitril und gegebenenfalls

in Gegenwart eines Säurebindemittels, wie z.B. Kaliumcarbonat, sowie gegebenenfalls in Gegenwart eines Katalysators, wie z.B. Natriumiodid, bei Temperaturen zwischen 0 und 100°C umsetzt.

Die Verbindungen der Formel (XI) sind allgemein bekannt.

Bei der Durchführung des erfindungsgemäßen Verfahrens (a) können als Säureakzeptoren alle üblichen Säurebindemittel Verwendung finden. Vorzugsweise in Frage kommen Alkalihydroxide, wie z.B. Natrium- und Kalium-hydroxid, Alkalicarbonate und -alkoholate, wie Natrium- und Kaliumcarbonat, Natrium- und Kaliummethylat bzw. -ethylat, ferner aliphatische, aromatische oder heterocyclische Amine, beispielsweise Triethylamin, Trimethylamin, Dimethylanilin, Dimethylbenzylamin, Pyridin, Diazabicylooctan, 1,5-Diazabicyclo-/4,3,0/-nonen-5 und 1,8-Diazabicyclo-/5,4,0/-undecen-7.

Als Verdünnungsmittel können bei der Durchführung des erfindungsgemäßen Verfahrens (a) praktisch alle inerten organischen Solventien verwendet werden. Vorzugsweise in Betracht kommen aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, ferner Ether, wie Diethylether und Dibutylether, Glycoldimethylether und Diglycoldimethylether, Tetrahydrofuran und Dioxan, außerdem Ketone, wie Aceton, Methylethyl-, Methyl-isopropyl- und Methyl-isobutylketon, Ester

wie Essigsäure-methylester und -ethylester, weiterhin Nitrile, z.B. Acetonitril und Propionnitril, darüber hinaus Amide, wie z.B. Dimethylformamid, Dimethylacetamid und N-Methyl-pyrrolidon, sowie stark polare Solventien wie Dimethylsulfoxid, Tetramethylensulfon und Hexamethylphosphorsäuretriamid.

Die Reaktionstemperaturen können bei der Durchführung des erfindungsgemäßen Verfahrens (a) innerhalb eines größeren Bereiches variiert werden. Im allgemeinen arbeitet man bei Temperaturen zwischen -20°C und +140°C, vorzugsweise zwischen 0°C und +80°C.

Das erfindungsgemäße Verfahren (a) wird ebenso wie die erfindungsgemäßen Verfahren (b) -(e) im allgemeinen unter Normaldruck ausgeführt.

Bei der Durchführung des erfindungsgemäßen Verfahrens (a) setzt man auf 1 Mol an Iminoester der Formel (II) 1 bis 4 Mol an Cyanamid der Formel (III) ein. Die Umsetzung wird im allgemeinen in einem geeigneten Verdünnungsmittel, gegebenenfalls in Gegenwart eines Säureakzeptors durchgeführt, und das Reaktionsgemisch wird mehrere Stunden bei der erforderlichen Temperatur gerührt. Die Aufarbeitung und die gegebenenfalls erforderliche Reinigung der Substanzen erfolgen nach üblichen Methoden.

Die bei der Durchführung des erfindungsgemäßen Verfahrens (b) als Ausgangsstoffe benötigten Phenoxy-phenol-Derivate sind durch die Formel (IV) definiert. In dieser

Formel haben $R^1$, $R^2$, $R^3$ und $R^4$ vorzugsweise diejenigen Bedeutungen, die bereits im Zusammenhang mit der Beschreibung der erfindungsgemäßen Stoffe der Formel (I) vorzugsweise für diese Reste genannt wurden. Z steht vorzugsweise für Wasserstoff, ein Natrium- oder Kaliumion oder für ein Äquivalent eines Calciumions.

Die Phenoxy-phenol-Derivate der Formel (IV) sind bekannt oder lassen sich nach bekannten Verfahren in einfacher Weise herstellen (vgl. US-PS 3 928 416).

Die bei dem erfindungsgemmäßen Verfahren (b) weiterhin als Ausgangsstoffe benötigten Verbindungen sind durch die Formel (V) definiert. In dieser Formel haben $R^5$, $R^6$ und X vorzugsweise diejenigen Bedeutungen, die bereits im Zusammenhang mit der Beschreibung der erfindungsgemäßen Stoffe der Formel (I) vorzugsweise für diese Reste genannt wurden. Hal steht für Chlor oder Brom.

Die Verbindungen der Formel (V) sind neu. Sie lassen sich herstellen, indem man Halogen-iminoester der Formel

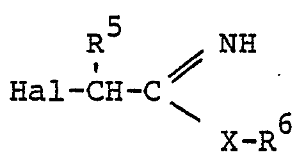

(XII)

in welcher

$R^5$, $R^6$, X und Hal die oben angegebene Bedeutung haben,

oder deren Hydrohalogenide, insbesondere Hydrochloride, mit Cyanamid in Gegenwart eines Verdünnungsmittels, wie zum Beispiel Toluol, sowie gegebenenfalls in Gegenwart eines Säurebindemittels bei Temperaturen zwischen -20°C und +120°C, vorzugsweise zwischen 0°C und 80°C umsetzt.

Die Halogen-iminoester der Formel (XII) sind bekannt oder lassen sich nach bekannten Methoden in einfacher Weise herstellen (vgl. DE-OS 2 746 957).

Als Säureakzeptoren können bei dem erfindungsgemäßen Verfahren (b) alle üblichen Säurebindemittel verwendet werden. Vorzugsweise in Frage kommen alle diejenigen Säurebinder, die im Zusammenhang mit der Beschreibung des erfindungsgemäßen Verfahrens (a) genannt wurden.

Als Verdünnungsmittel können bei der Durchführung des erfindungsgemäßen Verfahrens (b) praktisch alle inerten organischen Solventien verwendet werden. Vorzugsweise in Betracht kommen diejenigen Verdünnungsmittel, die im Zusammenhang mit der Beschreibung des erfindungsgemäßen Verfahrens (a) genannt wurden.

Die Reaktionstemperaturen können bei der Durchführung des erfindungsgemäßen Verfahrens (b) innerhalb eines größeren Bereiches variiert werden. Im allgemeinen arbeitet man bei Temperaturen zwischen -20 und +150°C, vorzugsweise zwischen 0 und +120°C.

Bei der Durchführung des erfindungsgemäßen Verfahrens (b) werden die Ausgangsstoffe der Formeln (IV) und (V) im allgemeinen in etwa äquimolaren Mengen eingesetzt. Ein Überschuß der einen oder anderen Komponente ist möglich. Die Umsetzung wird im allgemeinen in einem geeigneten Verdünnungsmittel, gegebenenfalls in Gegenwart eines Säureakzeptors durchgeführt, und das Reaktionsgemisch wird mehrere Stunden bei der erforderlichen Temperatur gerührt. Die Aufarbeitung und die gegebenenfalls erforderliche Reinigung der isolierten Substanzen erfolgen nach üblichen Methoden.

Die bei dem erfindungsgemäßen Verfahren (c) als Ausgangsstoffe benötigten Halogenbenzole sind durch die Formel (VI) definiert. In dieser Formel haben $R^1$, $R^2$ und $R^3$ vorzugsweise diejenigen Bedeutungen, die bereits im Zusammenhang mit der Beschreibung der erfindungsgemäßen Stoffe der Formel (I) vorzugsweise für diese Reste genannt wurden. Hal' steht für Chlor oder Brom.

Die Halogenbenzole der Formel (VI) sind bekannt oder lassen sich nach bekannten Methoden herstellen.

Die bei dem erfindungsgemäßen Verfahren (c) weiterhin als Ausgangsstoffe benötigten Phenol-Derivate sind durch die Formel (VII) definiert. In dieser Formel haben $R^4$, $R^5$, $R^6$ und X vorzugsweise diejenigen Bedeutungen, die bereits im Zusammenhang mit der Beschreibung der erfindungsgemäßen Stoffe der Formel (I) vorzugsweise für diese Reste genannt wurden. Z' steht vorzugsweise für

Wasserstoff, ein Natrium- oder Kaliumion oder für ein Äquivalent eines Calciumions.

Die Phenol-Derivate der Formel (VII) sind bisher noch nicht bekannt. Sie lassen sich jedoch analog zu dem erfindungsgemäßen Verfahren (b) herstellen. So erhält man Phenol-Derivate der Formel (VII), indem man Verbindungen der Formel

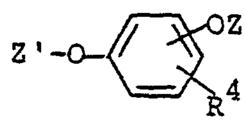

(XIII)

in welcher

$R^4$, Z und Z' die oben angegebene Bedeutung haben,

mit Verbindungen der Formel

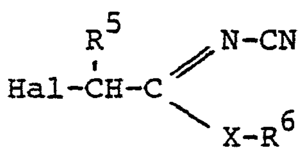

(V)

in welcher

$R^5$, $R^6$, X und Hal die oben angegebene Bedeutung haben,

gegebenenfalls in Gegenwart eines Säureakzeptors und gegebenenfalls in Gegenwart eines Verdünnungsmittels umsetzt.

Als Säureakzeptoren und Verdünnungsmittel kommen bei diesem Verfahren zur Herstellung der Phenol-Derivate der Formel (VII) alle diejenigen Säurebinder und Lösungsmittel in Frage, die bereits im Zusammenhang mit der Beschreibung des erfindungsgemäßen Verfahrens (a) genannt wurden. Die Reaktionsbedingungen und die Durchführung des Verfahrens entsprechen denen des Verfahrens (b).

Als Säureakzeptoren können bei dem erfindungsgemäßen Verfahren (c) alle üblichen Säurebindemittel verwendet werden. Vorzugsweise in Frage kommen alle diejenigen Säurebinder, die im Zusammenhang mit der Beschreibung des erfindungsgemäßen Verfahrens (a) genannt wurden.

Als Verdünnungsmittel können bei der Durchführung des erfindungsgemäßen Verfahrens (c) praktisch alle inerten organischen Solventien verwendet werden. Vorzugsweise in Betracht kommen diejenigen Verdünnungsmittel, die im Zusammenhang mit der Beschreibung des erfindungsgemäßen Verfahrens (a) genannt wurden.

Die Reaktionstemperaturen können bei der Durchführung des erfindungsgemäßen Verfahrens (c) innerhalb eines größeren Bereiches variiert werden. Im allgemeinen arbeitet man bei Temperaturen zwischen 0°C und 180°C, vorzugsweise zwischen 20°C und 140°C.

Bei der Durchführung des erfindungsgemäßen Verfahrens (c) werden die Ausgangsstoffe der Formeln (VI) und (VII) im allgemeinen in äquimolaren Mengen eingesetzt. Ein

Überschuß an der einen oder anderen Komponente ist möglich. Die Umsetzung wird im allgemeinen in einem geeigneten Verdünnungsmittel sowie gegebenenfalls in Gegenwart eines Säureakzeptors durchgeführt, und das Reaktionsgemisch wird mehrere Stunden bei der erforderlichen Temperatur gerührt. Die Aufarbeitung und die Reinigung der Substanzen erfolgen nach üblichen Methoden.

Die bei dem erfindungsgemäßen Verfahren (d) als Ausgangsstoffe benötigten Phenol-Derivate sind durch die Formel (VIII) definiert. In dieser Formel haben $R^1$, $R^2$ und $R^3$ vorzugsweise diejenigen Bedeutungen, die bereits im Zusammenhang mit der Beschreibung der erfindungsgemäßen Stoffe der Formel (I) vorzugsweise für diese Reste genannt wurden. $Z^{II}$ steht vorzugsweise für Wasserstoff, ein Natrium- oder Kaliumion oder für ein Äquivalent eines Calciumions.

Die Phenol-Derivate der Formel (VIII) sind bekannt oder lassen sich nach bekannten Methoden herstellen (vgl. US-PS 3 928 416 und DE-OS 2 333 848).

Die bei dem erfindungsgemäßen Verfahren (d) weiterhin als Ausgangsstoffe benötigten Verbindungen sind durch die Formel (IX) definiert.

In dieser Formel haben $R^5$, $R^6$ und X vorzugsweise diejenigen Bedeutungen, die bereits im Zusammenhang mit der Beschreibung der erfindungsgemäßen Stoffe der Formel (I) vorzugsweise für diese Reste genannt wurden. $Hal^{II}$ steht für Chlor oder Brom.

Die Verbindungen der Formel (IX) sind bisher noch nicht bekannt. Sie lassen sich analog zu den Verfahren (a), (b) und (d) herstellen, indem man Phenol-Derivate der Formel

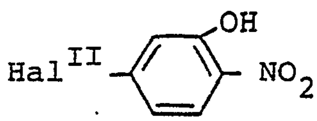

(XIV)

in welcher

$Hal^{II}$ die oben angegebene Bedeutung hat,

mit Verbindungen der Formel

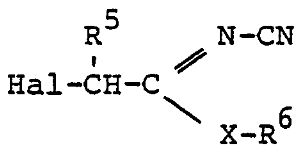

(V)

in welcher

$R^5$, $R^6$, X und Hal die oben angegebene Bedeutung haben,

gegebenenfalls in Gegenwart eines Säureakzeptors und gegebenenfalls in Gegenwart eines Verdünnungsmittels umsetzt.

Die Phenol-Derivate der Formel (XIV) sind bereits bekannt.

Als Säureakzeptoren und Verdünnungsmittel können bei dem Verfahren zur Herstellung der Verbindungen der Formel (IX) alle diejenigen Hilfsstoffe eingesetzt werden, die in diesem Zusammenhang bereits im Falle des erfindungsgemäßen Verfahrens (a) genannt wurden. Die Reaktionstemperaturen liegen im allgemeinen zwischen -20°C und +160°C, vorzugsweise zwischen 0°C und 120°C. Die Durchführung der Umsetzung und die Aufarbeitung erfolgen nach üblichen Methoden.

Als Säureakzeptoren können bei dem erfindungsgemäßen Verfahren (d) alle üblichen Säurebindemittel verwendet werden. Vorzugsweise in Frage kommen alle diejenigen Säurebinder, die im Zusammenhang mit der Beschreibung des erfindungsgemäßen Verfahrens (a) genannt wurden.

Als Verdünnungsmittel können bei der Durchführung des erfindungsgemäßen Verfahrens (d) praktisch alle inerten organischen Solventien verwendet werden. Vorzugsweise in Betracht kommen diejenigen Verdünnungsmittel, die im Zusammenhang mit der Beschreibung des erfindungsgemäßen Verfahrens (a) genannt wurden.

Die Reaktionstemperaturen können bei der Durchführung des erfindungsgemäßen Verfahrens (d) innerhalb eines größeren Bereiches variiert werden. Im allgemeinen arbeitet man bei Temperaturen zwischen 0°C und 180°C, vorzugsweise zwischen 20°C und 140°C.

Bei der Durchführung des erfindungsgemäßen Verfahrens (d) werden die Ausgangsstoffe der Formeln (VIII) und (IX) im allgemeinen in etwa äquimolaren Mengen eingesetzt. Ein Überschuß der einen oder anderen Komponente ist möglich. Die Umsetzung wird im allgemeinen in einem geeigneten Verdünnungsmittel sowie gegebenenfalls in Gegenwart eines Säureakzeptors durchgeführt, und das Reaktionsgemisch wird mehrere Stunden bei der erforderlichen Temperatur gerührt. Die Aufarbeitung und die Reinigung der Substanzen erfolgen nach üblichen Methoden.

Die bei der Durchführung des erfindungsgemäßen Verfahrens (e) als Ausgangsstoffe benötigten Verbindungen sind durch die Formel (Ia) definiert. In dieser Formel haben $R^1$, $R^2$, $R^3$, $R^5$, $R^6$ und X vorzugsweise diejenigen Bedeutungen, die bereits im Zusammenhang mit der Beschreibung der erfindungsgemäßen Stoffe der Formel (I) vorzugsweise für diese Reste genannt wurden.

Die Stoffe der Formel (Ia) sind neu; sie lassen sich jedoch nach dem erfindungsgemäßen Verfahren (a) in einfacher Weise herstellen.

Zur Nitrierung verwendet man im Falle des erfindungsgemäßen Verfahrens (e) vorzugsweise konzentrierte Salpetersäure.

Das erfindungsgemäße Verfahren (e) wird gegebenenfalls unter Verwendung eines Verdünnungsmittels durchgeführt. Als Verdünnungsmittel kommen hierbei vorzugsweise halogenierte Kohlenwasserstoffe, wie z.B. Methylenchlorid, in Betracht.

Das erfindungsgemäße Verfahren (e) wird gegebenenfalls in Gegenwart eines Katalysators durchgeführt. Als Katalysatoren kommen vorzugsweise Protonensäuren, wie z.B. Schwefelsäure oder Essigsäure, in Betracht.

Die Reaktionstemperatur wird bei Verfahren (e) im allgemeinen zwischen -20 und +140°C, vorzugsweise zwischen 0 und 100°C gehalten.

Zur Durchführung des erfindungsgemäßen Verfahrens (e) setzt man auf 1 Mol einer Verbindung mit der Formel (Ia) im allgemeinen 0,9 bis 2 Mol, vorzugsweise 1,0 bis 1,3 Mol an Nitrierungsmittel und gegebenenfalls etwa die gleiche Menge eines Katalysators ein. Die Ausgangskomponenten werden vorzugsweise unter Eiskühlung zusammengegeben und dann gegebenenfalls bei leicht erhöhter Temperatur bis zum Ende der Umsetzung gerührt.

Die Aufarbeitung erfolgt nach üblichen Methoden. Im allgemeinen verfährt man in der Weise, daß man das Reaktionsgemisch nach beendeter Umsetzung in Eiswasser gießt, absaugt und das anfallende Produkt gegebenenfalls durch Umkristallisieren reinigt.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale und selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst-, Ziergehölz-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe sind besonders gut zur selektiven Unkrautbekämpfung in verschiedenen wichtigen Kulturen, wie zum Beispiel Rüben und Sojabohnen, geeignet. Außerdem lassen sich die erfindungsgemäßen Wirkstoffe sehr gut zur Entlaubung und zur Austrocknung der Blätter bei Baumwolle verwenden.

Die Wirkstoffe können in die üblichen Formulierungen überführt werden, wie Lösungen, Emulsionen, Spritzpulver, Suspensionen, Granulate, Suspensions-Emulsions-Konzentrate, wirkstoffimprägnierte Natur- und synthetische Stoffe, Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzlische Öle, Alkohole, wie Butanol oder Gylcol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze, natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silicate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen

Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffe können als solche oder in ihren Formulierungen auch in Mischung mit bekannten Herbiziden oder Pflanzenwachstumsregulatoren zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4 (1H, 3H)-dion oder N-(2-Benzthiazolyl)-N,N'-dimethyl-harnstoff zur Unkrautbekämpfung in Getreide; 4-Amino-3-methyl-6-phenyl-1,2,4-triazin-5 (4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Die Anwendung wird vorzugsweise nach dem Auflaufen der Pflanzen, also im post-emergence-Verfahren, vorgenommen. Sie können auch vor der Saat in den Boden eingearbeitet werden.

Die angewandte Wirkstoffmenge kann in einem größeren Bereich schwanken. Sie hängt im wesentlichen ab von der Art des gewünschten Effektes ab. Im allgemeinen liegen die Aufwandmengen zwischen 0,01 und 20 kg Wirkstoff pro Hektar Bodenfläche, vorzugsweise zwischen 0,05 und 10 kg pro ha.

Die Herstellung und die Verwendung der erfindungsgemäßen Wirkstoffe geht aus den nachfolgenden Beispielen hervor.

## Herstellungsbeispiele

### Beispiel 1

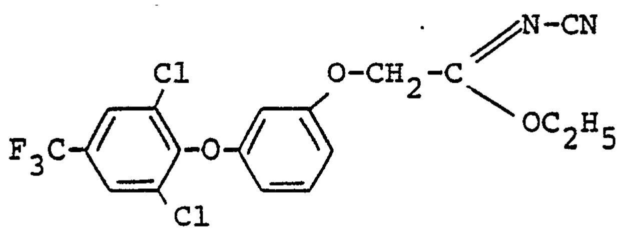

In eine Suspension von 14,7 g (0,033 Mol) α-[3-(2,6-Dichlor-4-trifluormethyl-phenoxy)-phenoxy]-iminoessigsäure-ethylester-hydrochlorid in 100 ml Toluol werden bei Raumtemperatur unter Rühren 4,2 g (0,1 Mol) Cyanamid eingetragen. Man rührt nach beendeter Zugabe noch weitere 8 Stunden und läßt dann über Nacht stehen unter Ausschluß von Wasser. Anschließend wird aufgearbeitet, indem man das Reaktionsgemisch zweimal mit je 100 ml Wasser wäscht, die organische Phase über Natriumsulfat trocknet und einengt. Es verbleibt ein öliges Produkt, das allmählich kristallisiert. Man verreibt mit Diisopropylether, saugt ab und trocknet. Auf diese Weise erhält man 5,5 g (38,5 % der Theorie) an α-[3-(2,6-Dichlor-4-trifluormethyl-phenoxy)-phenoxy]-cyanimino-essigsäure-ethylester mit einem Schmelzpunkt von 68°C.

### Vorprodukte

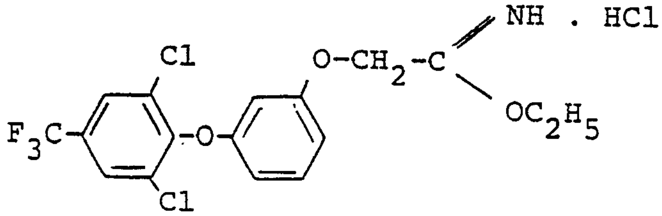

(II-1)

Eine Lösung von 12 g (0,033 Mol) /3-(2,6-Dichlor-4-trifluormethyl-phenoxy)-phenoxy7-essigsäurenitril in 100 ml Toluol und 1,8 g (0,039 Mol) absolutem Ethanol wird bei 0°C mit wasserfreiem Chlorwasserstoff-Gas gesättigt. Nach 14-stündigem Stehenlassen wird das Reaktionsgemisch unter vermindertem Druck eingeengt. Es verbleiben 14,7 g an rohem /3-(2,6-Dichlor-4-trifluormethyl-phenoxy)-phenoxy7-iminoessigsäure-ethylester-hydrochlorid, das ohne weitere Reinigung für die folgenden Umsetzungen verwendet wird. Das mit Cyclohexan verriebene und auf Ton abgepreßte Rohprodukt besitzt einen Schmelzpunkt von 93 - 94°C.

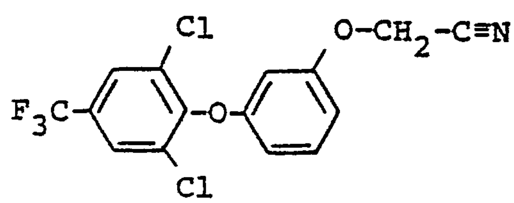

(X-1)

Ein Gemisch aus 32 g (0,1 Mol) 2,6-Dichlor-4-trifluormethyl-3'-hydroxy-diphenylether, 100 ml Acetonitril, 1 g Natriumjodid und 15 g Kaliumcarbonat wird unter Feuchtigkeitsausschluß zum Sieden erhitzt und dann innerhalb von 30 Minuten mit 10 g (0,13 Mol) Chloracetonitril versetzt. Man kocht weitere 20 Stunden unter Rückfluß, filtriert dann und engt das Reaktionsgemisch durch Abziehen des Lösungsmittels ein. Das verbleibende ölige

Produkt wird durch kurzes Erhitzen im Hochvakuum von noch enthaltenen flüchtigen Anteilen befreit. Man erhält auf diese Weise 34 g (95 % der Theorie) an /3-(2,6-Dichlor-4-trifluormethyl-phenoxy)-phenoxy7-essigsäurenitril.

Beispiel 2

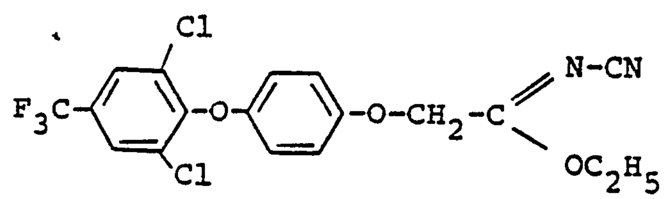

In eine Suspension von 14,7 g (0,33 Mol) α-/4-(2,6-Dichlor-4-trifluormethyl-phenoxy)-phenoxy7-iminoessigsäureethylester-hydrochlorid in 100 ml Toluol werden bei Raumtemperatur unter Rühren 4,2 g (0,1 Mol) Cyanamid eingetragen. Man rührt nach beendeter Zugabe noch weitere 8 Stunden und läßt dann über Nacht stehen unter Ausschluß von Wasser. Anschließend wird aufgearbeitet, indem man das Reaktionsgemisch zweimal mit je 100 ml Wasser wäscht, die organische Phase über Natriumsulfat trocknet und einengt. Es verbleibt ein öliges Produkt, das chromatographisch gereinigt wird (Säule: 50 cm lang, Ø 4 cm; Trägermaterial: Kieselgel, Laufmittel: Cyclohexan/Aceton steigender Polarität). Nach dem Eindampfen des Eluates verbleibt ein Produkt, das allmählich kristallisiert. Durch Verreiben dieses kristallinen Stoffes mit Diisopropylether, Absaugen und Trocknen erhält man 1,2 g (8,4 % der Theorie) an α-/4-(2,6-Dichlor-4-trifluormethyl-phenoxy)-phenoxy7-cyanimino-essigsäure-ethylester mit einem Schmelzpunkz von 88°C.

Vorprodukte

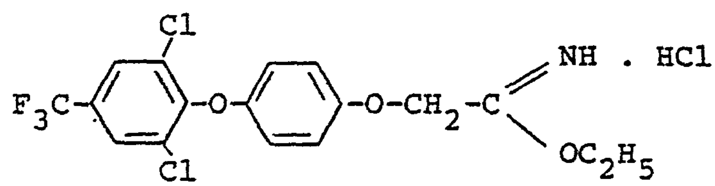

(II-2)

Ein Gemisch aus 12 g (0,033 Mol) [4-(2,6-Dichlor-4-trifluormethyl-phenoxy)-phenoxy]-essigsäure-nitril, 100 ml Toluol und 1,8 g (0,039 Mol) Ethanol wird auf 0°C abgekühlt und mit Chlorwasserstoff-Gas gesättigt. Man läßt über Nacht stehen und engt dann durch Abziehen des Lösungsmittel ein. Auf diese Weise erhält man 14,7 g (100 % der Theorie) an α-[4-(2,6-Dichlor-4-trifluormethyl-phenoxy)-phenoxy]-imino-essigsäure-ethylester-hydrochlorid in Form eines kristallinen Produktes vom Schmelzpunkt 96 - 98°C.

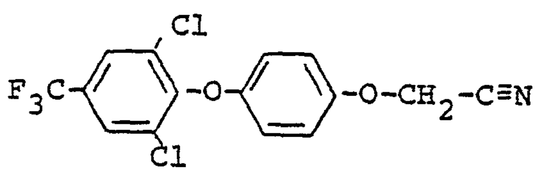

(X-2)

Ein Gemisch aus 16 g (0,05 Mol) 2,6-Dichlor-4-trifluormethyl-4'-hydroxy-diphenylether, 100 ml Acetonitril, 1 g Natriumjodid und 8,5 g Kaliumcarbonat wird unter Feuchtigkeitsausschluß zum Sieden erhitzt und dann innerhalb von 30 Minuten mit 6 g (0,08 Mol) Chloracetonitril versetzt. Man kocht weitere 20 Stunden unter Rückfluß, saugt dann ab und engt das Reaktionsgemisch durch Abziehen des Lösungsmittels ein. Das verbleibende ölige Produkt wird durch kurzes Erhitzen im Hochvakuum von noch enthaltenen flüchtigen Anteilen befreit. Man nimmt den Rückstand in Chloroform auf, wäscht mit Wasser, trocknet und engt erneut ein. Auf diese Weise erhält man 15 g (84 % der Theorie) an /4-(2,6-Dichlor-4-trifluormetyl-phenoxy)-phenoxy/-essigsäure-nitril.

Die in den folgenden Beispielen aufgeführten Verbindungen wurden ebenfalls nach den vorstehend beschriebenen Methoden hergestellt:

Beispiel 3

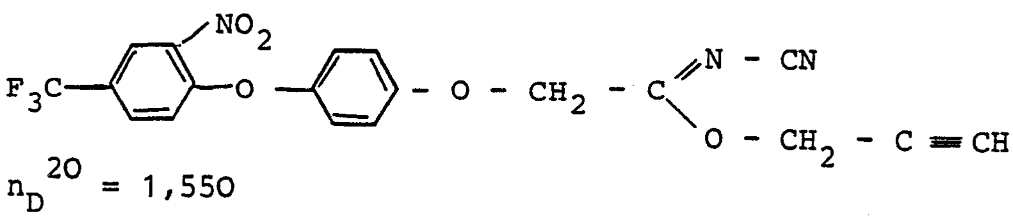

$n_D^{20} = 1,550$

Beispiel 4

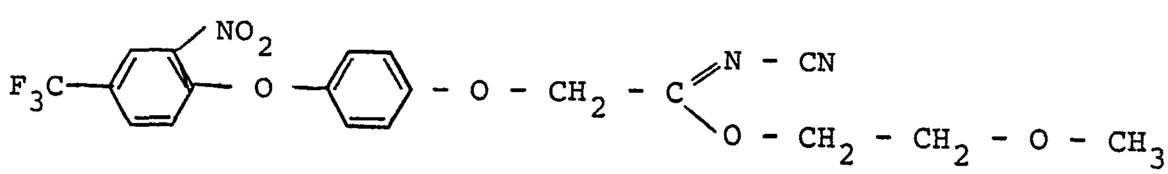

Viskoses Öl.

IR-Spektrum: 2200 $cm^{-1}$ (C ≡ N)

1535 $cm^{-1}$ ($NO_2$)

Beispiel A

Post-emergence-Test

Lösungsmittel: 5 Gewichtsteile Aceton
Emulgator: 1 Gewichtsteil Alkylarylpolyglycolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

In diesem Test zeigen die erfindungsgemäßen Wirkstoffe eine sehr gute herbizide Wirksamkeit.

Beispiel B

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffes pro Flächeneinheit. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)
100 % = totale Vernichtung

In diesem Test zeigen die erfindungsgemäßen Wirkstoffe eine sehr gute herbizide Wirksamkeit.

Patentansprüche

1) Substituierte Diphenylether der Formel

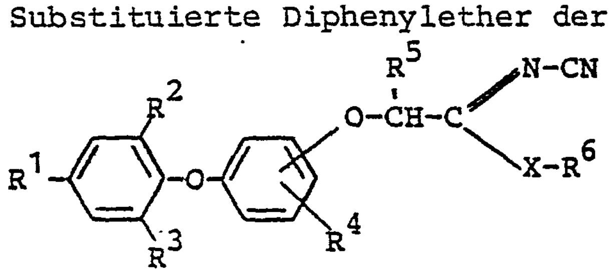

(I)

in welcher

$R^1$ für Halogen oder Trifluormethyl steht,

$R^2$ für Wasserstoff, Halogen, Trifluormethyl oder Nitro steht,

$R^3$ für Wasserstoff oder Halogen steht,

$R^4$ für Wasserstoff, Halogen oder Nitro steht,

$R^5$ für Wasserstoff oder Alkyl mit 1 bis 5 Kohlenstoffatomen steht,

$R^6$ für Alkyl, Alkenyl, Alkinyl oder Alkoxyalkyl steht und

X für Sauerstoff oder Schwefel steht.

2) Substituierte Diphenylether der Formel (I) in denen

$R^1$ für Fluor, Chlor, Brom oder Trifluormethyl, steht,

$R^2$ für Wasserstoff, Fluor, Chlor, Brom, Trifluormethyl oder Nitro steht,

$R^3$ für Wasserstoff, Fluor, Chlor oder Brom, steht,

$R^4$ für Wasserstoff, Fluor, Chlor, Brom oder Nitro, steht,

$R^5$ für Wasserstoff oder Alkyl mit 1 bis 4 Kohlenstoffatomen, steht,

$R^6$ für Alkyl mit 1 bis 6 Kohlenstoffatomen, Alkenyl mit 2 bis 6 Kohlenstoffatomen, Alkinyl mit 2 bis 6 Kohlenstoffatomen, Alkoxyalkyl mit 1 bis 4 Kohlenstoffatomen im Alkoxyteil und 1 bis 4 Kohlenstoffatomen im Alkylteil steht und

X für Sauerstoff oder Schwefel steht.

3) Verfahren zur Herstellung von substituierten Diphenylethern der Formel

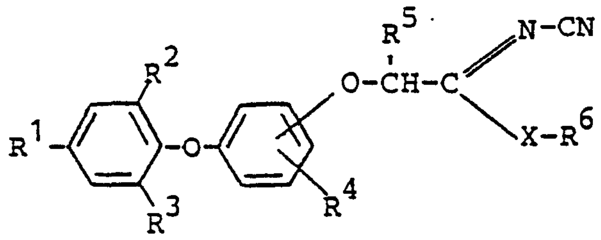

(I)

in welcher

$R^1$ für Halogen oder Trifluormethyl steht,

$R^2$ für Wasserstoff, Halogen, Trifluormethyl oder Nitro steht,

$R^3$ für Wasserstoff oder Halogen steht,

$R^4$ für Wasserstoff, Halogen oder Nitro steht,

$R^5$ für Wasserstoff oder Alkyl mit 1 bis 5 Kohlenstoffatomen steht,

$R^6$ für Alkyl, Alkenyl, Alkinyl oder Alkoxyalkyl steht und

X für Sauerstoff oder Schwefel steht,

dadurch gekennzeichnet, daß man

a) Iminoester der Formel

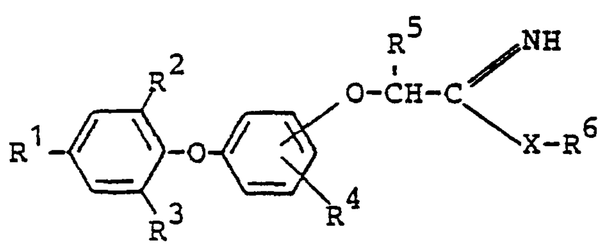

(II)

in welcher

$R^1$, $R^2$, $R^3$, $R^4$, $R^5$, $R^6$ und X die oben angegebene Bedeutung haben,

oder Hydrohalogenide der Iminoester der Formel (II) mit Cyanamid der Formel

$H_2N-CN$ (III)

gegebenenfalls in Gegenwart eines Säureakzeptors und gegebenenfalls in Gegenwart eines Verdünnungsmittels umsetzt,

oder

b) Phenoxy-phenol-Derivate der Formel

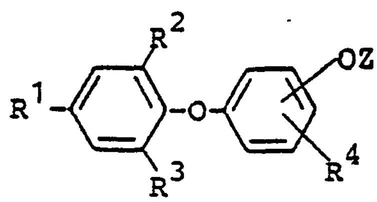

(IV)

in welcher

$R^1$, $R^2$, $R^3$ und $R^4$ die oben angegebene Bedeutung haben und

Z für Wasserstoff oder ein Äquivalent eines Alkalimetalles oder Erdalkalimetalles steht,

mit Verbindungen der Formel

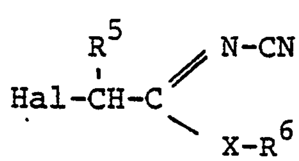

(V)

in welcher

$R^5$, $R^6$ und X die oben angegebene Bedeutung haben und

Hal für Chlor oder Brom steht,

gegebenenfalls in Gegenwart eines Säureakzeptors und gegebenenfalls in Gegenwart eines Verdünnungsmittels umsetzt,

oder

c) Halogenbenzole der Formel

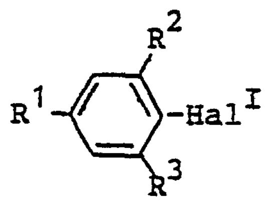

(VI)

in welcher

$R^1$, $R^2$ und $R^3$ die oben angegebene Bedeutung haben und

$Hal^I$ für Chlor oder Brom steht,

mit Phenol-Derivaten der Formel

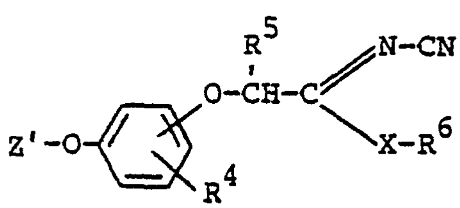

(VII)

in welcher

$R^4$, $R^5$, $R^6$ und X die oben angegebene Bedeutung haben und

Z' für Wasserstoff oder ein Äquivalent eines Alkalimetalles oder eines Erdalkalimetalles steht,

gegebenenfalls in Gegenwart eines Säureakzeptors und gegebenenfalls in Gegenwart eines Verdünnungsmittels umsetzt,

oder

d) Phenol-Derivate der Formel

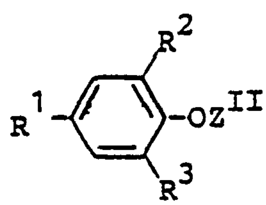

(VIII)

in welcher

$R^1$, $R^2$ und $R^3$ die oben angegebene Bedeutung haben und

$Z^{II}$ für Wasserstoff oder ein Äquivalent eines Alkalimetalles oder eines Erdalkalimetalles steht,

mit Verbindungen der Formel

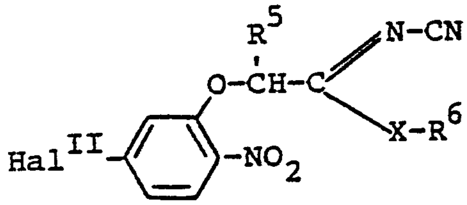

(IX)

in welcher

$R^5$, $R^6$ und X die oben angegebene Bedeutung haben und

$Hal^{II}$ für Chlor oder Brom steht,

gegebenenfalls in Gegenwart eines Säureakzeptors und gegebenenfalls in Gegenwart eines Verdünnungsmittels umsetzt,

oder

e) Verbindungen der Formel

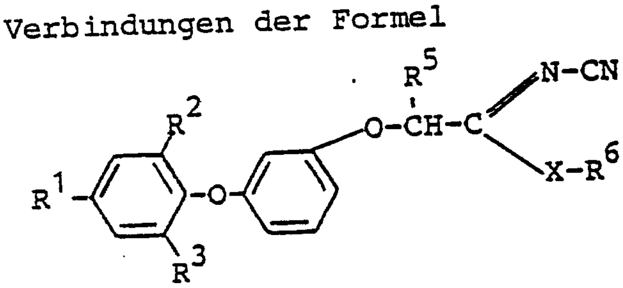

(Ia)

in welcher

$R^1$, $R^2$, $R^3$, $R^5$, $R^6$ und X die oben angegebene Bedeutung haben,

mit einem Nitrierungsmittel, gegebenenfalls in Gegenwart eines Katalysators sowie gegebenenfalls in Gegenwart eines Verdünnungsmittels umsetzt.

4) Herbizide Mittel, gekennzeichnet durch einen Gehalt an mindestens einem substituierten Diphenylether der Formel (I).

5) Verwendung von substituierten Diphenylethern der Formel (I) zur Bekämpfung von Unkräutern.

6) Verbindungen der Formel

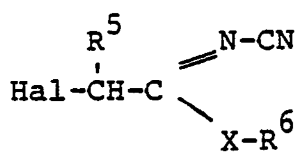

(V)

in welcher

$R^5$ für Wasserstoff oder Alkyl mit 1 bis 5 Kohlenstoffatomen steht,

$R^6$ für Alkyl, Alkenyl, Alkinyl oder Alkoxyalkyl steht,

X für Sauerstoff oder Schwefel steht und

Hal für Chlor oder Brom steht.

7) Verfahren zur Herstellung von Verbindungen der Formel

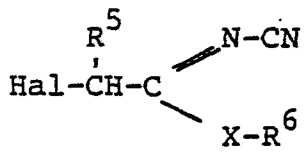

(V)

in welcher

$R^5$ für Wasserstoff oder Alkyl mit 1 bis 5 Kohlenstoffatomen steht,

$R^6$ für Alkyl, Alkenyl, Alkinyl oder Alkoxyalkyl steht,

X für Sauerstoff oder Schwefel steht und

Hal für Chlor oder Brom steht,

dadurch gekennzeichnet, daß man

Halogen-iminoester der Formel

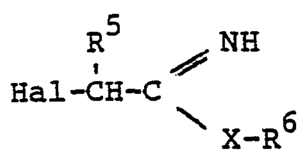

(XII)

in welcher

$R^5$, $R^6$, X und Hal die oben angegebene Bedeutung haben,

oder deren Hydrohalogenide mit Cyanamid in Gegenwart eines Verdünnungsmittels sowie gegebenenfalls in Gegenwart eines Säurebindemittels umsetzt.

8) Phenol-Derivate der Formel

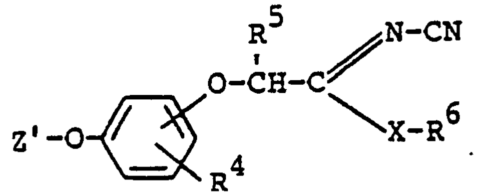

(VII)

in welcher

$R^4$ für Wasserstoff, Halogen oder Nitro steht,

$R^5$ für Wasserstoff oder Alkyl mit 1 bis 5 Kohlenstoffatomen steht,

$R^6$ für Alkyl, Alkenyl, Alkinyl oder Alkoxyalkyl steht,

X für Sauerstoff oder Schwefel steht und

Z' für Wasserstoff oder ein Äquivalent eines Alkalimetalles oder eines Erdalkalimetalles steht.

9) Verfahren zur Herstellung von Phenol-Derivaten der Formel

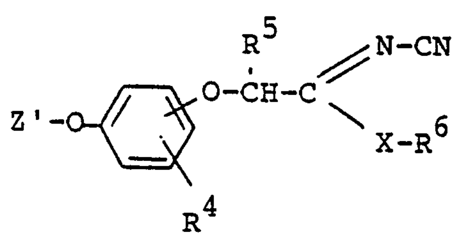

(VII)

in welcher

$R^4$ für Wasserstoff, Halogen oder Nitro steht,

$R^5$ für Wasserstoff oder Alkyl mit 1 bis 5 Kohlenstoffatomen steht,

$R^6$ für Alkyl, Alkenyl, Alkinyl oder Alkoxyalkyl steht,

X für Sauerstoff oder Schwefel steht und

Z' für Wasserstoff oder ein Äquivalent eines Alkalimetalles oder eines Erdalkalimetalles steht,

dadurch gekennzeichnet, daß man

Verbindungen der Formel

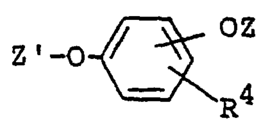

(XIII)

in welcher

$R^4$ und Z' die oben angegebene Bedeutung haben, und

Z für Wasserstoff oder ein Äquivalent eines Alkalimetalles oder Erdalkalimetalles steht,

mit Verbindungen der Formel

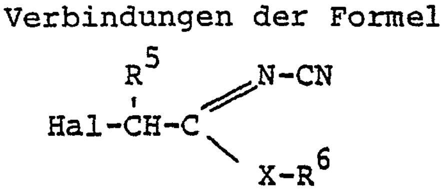

(V)

in welcher

$R^5$, $R^6$ und X die oben angegebene Bedeutung haben und

Hal für Chlor oder Brom steht,

gegebenenfalls in Gegenwart eines Säureakzeptors und gegebenenfalls in Gegenwart eines Verdünnungsmittels umsetzt.

10) Verbindungen der Formel

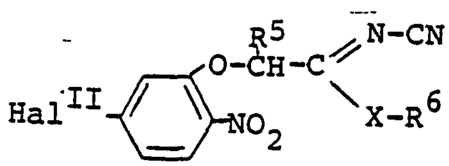

(IX)

in welcher

$R^5$ für Wasserstoff oder Alkyl mit 1 bis 5 Kohlenstoffatomen steht,

$R^6$ für Alkyl, Alkenyl, Alkinyl oder Alkoxyalkyl steht,

X für Sauerstoff oder Schwefel steht, und

$Hal^{II}$ für Chlor oder Brom steht.

11) Verfahren zur Herstellung von Verbindungen der Formel

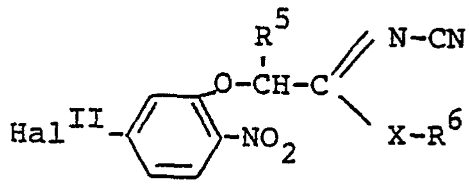

(IX)

in welcher

$R^5$ für Wasserstoff oder Alkyl mit 1 bis 5 Kohlenstoffatomen steht,

$R^6$ für Alkyl, Alkenyl, Alkinyl oder Alkoxyalkyl steht,

X für Sauerstoff oder Schwefel steht, und

$Hal^{II}$ für Chlor oder Brom steht,

dadurch gekennzeichnet, daß man Phenol-Derivate der Formel

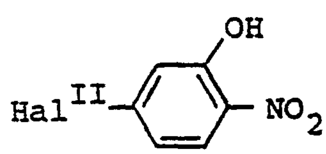

(XIV)

in welcher

$Hal^{II}$ die oben angegebene Bedeutung hat,

mit Verbindungen der Formel

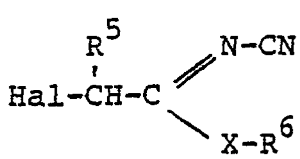

(V)

in welcher

$R^5$, $R^6$, X und Hal die oben angegebene Bedeutung haben,

gegebenenfalls in Gegenwart eines Säureakzeptors und gegebenenfalls in Gegenwart eines Verdünnungsmittels umsetzt.

0100425

Europäisches Patentamt

# EUROPÄISCHER RECHERCHENBERICHT

Nummer der Anmeldung

EP 83 10 6072

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| X | US-A-3 225 077 (SCHAEFER et al.) * Spalte 1, Zeile 27 * --- | 6,7 | C 07 C 125/08<br>A 01 N 47/40 |
| D,A | DE-A-2 613 697 (HOECHST AG) * Herstellungsbeispiele, Verfahren 1 * --- | 1-5 | |
| A | EP-A-0 034 883 (MOBIL OIL CORP.) * Seite 13, Zeile 4 * --- | 1-5 | |
| A | DE-A-2 835 695 (YAMANOUCHI PHARMACEUTICAL CO.) * Beispiel 2; Seite 16, Zeile 6 * ----- | 1-3,8-11 | |

RECHERCHIERTE SACHGEBIETE (Int. Cl. 3)

A 01 N 47/40
C 07 C 121/84
C 07 C 125/08

Der vorliegende Recherchenbericht wurde für alle Patentansprüche erstellt.

| Recherchenort | Abschlußdatum der Recherche | Prüfer |
|---|---|---|
| BERLIN | 07-09-1983 | BREW C.H. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82